(19) Europäisches Patentamt
European Patent Office
Office européen des brevets

(11) **EP 4 636 384 A1**

(12) **EUROPEAN PATENT APPLICATION**
published in accordance with Art. 153(4) EPC

(43) Date of publication:
**22.10.2025 Bulletin 2025/43**

(21) Application number: **23903859.9**

(22) Date of filing: **01.12.2023**

(51) International Patent Classification (IPC):
***G01N 7/04*** (2006.01)    ***G01N 1/40*** (2006.01)
***G06N 20/00*** (2019.01)

(52) Cooperative Patent Classification (CPC):
**G01N 1/40; G01N 7/04; G06N 20/00**

(86) International application number:
**PCT/KR2023/019732**

(87) International publication number:
**WO 2024/128656 (20.06.2024 Gazette 2024/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.12.2022 KR 20220176061**

(71) Applicants:
• **POSCO Holdings Inc.**
  **Pohang-si, Gyeongsangbuk-do 37859 (KR)**
• **RESEARCH INSTITUTE OF INDUSTRIAL SCIENCE & TECHNOLOGY**
  **Pohang-si, Gyeongsangbuk-do 37673 (KR)**

(72) Inventors:
• **YANG, Heok**
  **Pohang-si, Gyeongsangbuk-do 37655 (KR)**
• **PARK, Jae Sin**
  **Pohang-si, Gyeongsangbuk-do 37673 (KR)**
• **PARK, Woonkyoung**
  **Pohang-si, Gyeongsangbuk-do 37834 (KR)**
• **KUK, Seung Taek**
  **Pohang-si, Gyeongsangbuk-do 37671 (KR)**
• **KANG, Hee-Soo**
  **Pohang-si, Gyeongsangbuk-do 37673 (KR)**

(74) Representative: **Pfenning, Meinig & Partner mbB**
**Patent- und Rechtsanwälte**
**Theresienhöhe 11a**
**80339 München (DE)**

(54) **BRINE CONCENTRATING PROCESS PREDICTION SYSTEM**

(57)    Exemplary embodiments may provide a brine concentration process prediction system, the brine concentration process prediction system including: a data collection unit collecting data of an initial brine and a high-concentration brine; a data preprocessing unit converting the data collected by the data collection unit to be applied to a data processing unit provided at a later stage; a data processing unit calculating a solubility of each precipitate component using the data converted by the data preprocessing unit; and a data prediction unit predicting a final amount of precipitates and a concentration of each ion component in a final concentrated brine using the solubility of each precipitate component calculated by the data processing unit.

FIG. 1

```
DATA COLLECTION UNIT        ~10
        |
DATA PREPROCESSING UNIT     ~20
        |
DATA PROCESSING UNIT        ~30
        |
DATA PREDICTION UNIT        ~40
```

EP 4 636 384 A1

**Description**

**CROSS-REFERENCE TO RELATED APPLICATION**

This application claims priority to and the benefit of Korean Patent Application No. 10-2022-0176061 filed in the Korean Intellectual Property Office on December 15, 2022, the entire contents of which are incorporated herein by reference.

## BACKGROUND OF THE INVENTION

### (a) Field of the Invention

Exemplary embodiments of the present invention relate to a system for predicting the type of precipitates, the amount of precipitates, and concentrations of components in brine during a brine concentration process.

### (b) Description of the Related Art

Lithium (Li), which is element number 3, is the smallest element except hydrogen (number 1) and helium (number 2), which are gases at room temperature, is the lightest metal element, and has unique characteristics that cannot be replaced by other elements. Because of these characteristics, Li plays a key role in high-performance lithium-ion batteries, and a demand for Li is continuously increasing as the use of lithium-ion batteries increases in a mobile phone, an electric vehicle, and the like. Most of the lithium on Earth is in seawater, but the concentration of lithium in seawater is too low to be extracted commercially. In order to produce a lithium compound, lithium should be extracted from minerals containing lithium or extracted from brine with a high content of lithium. The most common method for extracting lithium from brine containing a high concentration of sodium (Na), potassium (K), magnesium (Mg), or calcium (Ca) is a method of trapping brine in a large-scale pond and concentrating the trapped brine through natural evaporation in the same way as a salt farm to prepare a high-concentration brine, chemically purifying impurities to prepare a lithium-containing solution having a high concentration of lithium and a low content of other elements, and then extracting the lithium-containing solution in the form of a solid lithium compound.

At this time, in the process of concentrating the brine from a low concentration to a high concentration, precipitation occurs as various ions in the brine are concentrated beyond their solubility, and a representative precipitate is sodium chloride (NaCl). In the case of Atacama salt lake in Chile, the most famous brine resource for producing lithium, a concentration of Li in the pond is increased by more than 20 to 30 times, and assuming that the concentration of Li is concentrated 20 times, 95% of the initially existing water is evaporated. This means that when the brine is evaporated by 19 from 20 while maintaining the remaining brine by only 1, the concentration is increased 20 times, and in this process, various salts equivalent to about 30 to 40% of the mass of the evaporated water are precipitated.

When substances containing lithium precipitate among these salts, a process loss occurs. Even when lithium is not present in the precipitated salt itself, lithium precipitates in the pond, and thus, the precipitated salt is in a state of being wet with the brine. Therefore, a certain amount of concentrated brine cannot be transferred to a post-process. In addition, a loss of lithium occurs during a brine concentration process because lithium leaks or is lost to the bottom of the pond. In general cases, a yield calculation in the brine concentration process is important because the loss of lithium during concentration is greater than the loss of lithium during purification of impurities in the high-concentration brine.

The most necessary information to predict the yield in the brine concentration process and the composition introduced into the impurity purification process after concentration is the type and amount of precipitates. Evaporation of water does not cause the loss of lithium, and in general cases, the amount of lithium lost is not large, and most lithium loss occurs in precipitates and water content.

Therefore, in order to predict the content of each element during the brine concentration process, there is a need to develop a system for predicting the type of precipitates, the amount of precipitates, and concentrations of components in brine with improved reliability when a concentration of each element increases due to evaporation.

## SUMMARY OF THE INVENTION

The present invention has been made in an effort to provide a system for predicting a composition of precipitates, the amount of precipitates, and a concentration of each component in concentrated brine during a brine concentration process.

An exemplary embodiment of the present invention provides a brine concentration process prediction system, the brine concentration process prediction system including: a data collection unit collecting data of an initial brine and a high-concentration brine; a data preprocessing unit converting the data collected by the data collection unit to be applied to a data processing unit provided at a later stage; a data processing unit calculating a solubility of each precipitate component using the data converted by the data preprocessing unit; and a data prediction unit predicting a final amount of precipitates and a concentration of each ion component in a final concentrated brine using the solubility of each precipitate component calculated by the data processing unit.

The data preprocessing unit may calculate a difference in concentration values of each ion component in the high-concentration brine and the initial brine collected by the data collection unit

The data processing unit calculating the solubility of each precipitate component using the data converted by the data preprocessing unit may calculate a solubility of each precipitate component through multiple non-linear regression analysis or machine learning.

The data processing unit may additionally calculate the amount of carbon dioxide absorbed, and the amount of carbon dioxide absorbed may be calculated using concentrations of calcium (Ca), sulfuric acid ($SO_4$), and boron (B) in the initial brine and the high-concentration brine and a pH value.

The data preprocessing unit, the data processing unit, and the data prediction unit may repeatedly perform calculations according to a change in data of the high-concentration brine collected by the data collection unit to calculate an optimal concentration of each ion component in a final concentrated brine.

A concentration of Li in the final concentrated brine may be 3.5 g/L to 18 g/L, and an error range of the final amount of precipitates predicted by the data prediction unit may be 5% or less.

According to an exemplary embodiment of the present invention, the type and amount of precipitated salts and the contents of components in concentrated brine may be predicted from changes in concentration of components in brine, and the operating conditions of the brine concentration process may be controlled to increase lithium production.

According to an exemplary embodiment of the present invention, the type and amount of precipitated salts and the contents of components in concentrated brine may be predicted from changes in concentration of components in brine, and the brine concentration process prediction system may be used to design an evaporation and concentration process in salt lake areas where quantitative prediction is difficult.

## BRIEF DESCRIPTION OF THE DRAWINGS

FIG. 1 is a schematic view illustrating a brine concentration process prediction system according to an exemplary embodiment of the present invention.

FIGS. 2A and 2B are schematic views illustrating a method of calculating component concentrations during a brine concentration process.

FIG. 3 is a diagram illustrating an activity coefficient and an ionic strength according to the existing thermodynamic calculation formula.

FIG. 4 is a diagram illustrating results according to Experimental Example 1 and Comparative Example 1.

FIG. 5 is a diagram illustrating results according to Example 1 and Experimental Example 1.

FIG. 6 is a diagram illustrating results according to Example 2 and Experimental Example 1.

FIG. 7 is a diagram illustrating results according to Example 3 and Experimental Example 2.

## DETAILED DESCRIPTION OF THE EMBODIMENTS

In the description of the present invention, the terms "first", "second", "third", and the like are used to describe various parts, components, regions, layers, and/or sections, but are not limited thereto. These terms are only used to differentiate a specific part, component, region, layer, or section from another part, component, region, layer, or section. Accordingly, a first component, part, region, layer, or section which will be described hereinafter may be referred to as a second component, part, region, layer, or section without departing from the scope of the present invention.

Terminologies used herein are to mention only a specific exemplary embodiment, and are not to limit the present invention. Singular forms used herein include plural forms as long as phrases do not clearly indicate an opposite meaning. The term "comprising" used in the specification concretely indicates specific properties, regions, integers, steps, operations, elements, and/or components, and is not to exclude the presence or addition of other specific properties, regions, integers, steps, operations, elements, and/or components.

Unless defined otherwise, all terms including technical terms and scientific terms used herein have the same meanings as understood by those skilled in the art to which the present invention pertains. Terms defined in a generally used dictionary are additionally interpreted as having the meanings matched to the related technical document and the currently disclosed contents, and are not interpreted as ideal or very formal meanings unless otherwise defined.

Hereinafter, exemplary embodiments of the present invention will be described in detail. However, these exemplary embodiments are provided as examples, and the present invention is not limited by these exemplary embodiments and is defined by only the scope of the claims to be described below.

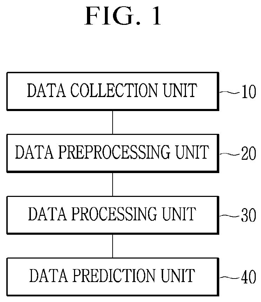

FIG. 1 is a schematic view illustrating a brine concentration process prediction system according to an exemplary embodiment of the present invention.

Referring to FIG. 1, the brine concentration process prediction system according to an exemplary embodiment of the present invention may include a data collection unit 10, a data preprocessing unit 20, a data processing unit 30, and a data prediction unit 40.

The data collection unit 10 may collect various data such as the type of components in an initial brine and a high-concentration brine, component concentration data, a composition of precipitates, the amount of precipitates, weather data, and a pH value of brine. The data collection unit 10 may build a database to store the collected data.

As an example, the following already measured data may be input into the data collection unit 10.

- Ion concentration in brine

[Table 1]

| Li (g/L) | SO$_4$ (g/L) | Ca (g/L) | Mg (g/L) | B (g/L) | K (g/L) | Na (g/L) |
|---|---|---|---|---|---|---|
| 1.702 | 3.793 | 2.324 | 0.031 | 0.581 | 17.91 | 101.88 |
| 1.857 | 3.763 | 2.383 | 0.049 | 0.633 | 19.35 | 106.98 |
| 2.894 | 5.126 | 3.063 | 0.113 | 0.824 | 28.78 | 101.03 |
| 3.412 | 5.357 | 3.119 | 0.133 | 0.848 | 33.99 | 97.80 |
| 5.046 | 4.039 | 2.526 | 0.196 | 0.925 | 0.05 | 87.35 |

The data collected by the data collection unit 10 may be transmitted to the data preprocessing unit 20. The data preprocessing unit 20 may calculate a difference in concentration values of each ion component in the high-concentration brine and the initial brine, and may transmit the calculated difference in concentration values of each ion component to the data processing unit 30.

As an example, the data preprocessing unit 20 may perform the following calculations using data such as the ion concentration in the brine and the pH.

1) Anion (OH$^-$, Cl$^-$) concentrations that cannot be obtained by the data collection unit

$\rightarrow$ Calculate a concentration of OH$^-$ using pH (using phreeqc thermodynamics program)
$\rightarrow$ Calculate a concentration of Cl$^-$ considering a charge balance

2) Calculate a ratio of B(OH)$_3$:B(OH)$_4^-$ of the ion represented by B

$\rightarrow$ Calculate the ratio using an equilibrium constant of the reaction B(OH)$_3$ + OH$^-$ $\rightarrow$ B(OH)$_4^-$ since the pH is fixed
$\rightarrow$ Recalculate the concentration of Cl$^-$ ions since the amount of all cations and anions have been calculated

Through the above series of calculations, the data preprocessing unit 20 may obtain the following data.

[Table 2]

| | | | | | | | | (Unit of concentration: mol/L) | |
|---|---|---|---|---|---|---|---|---|---|
| Li$^+$ | SO$_4^{2-}$ | Ca$^{2+}$ | Mg$^{2+}$ | B(OH) 3 | B(OH) $^{4-}$ | K$^+$ | Na$^+$ | Cl$^-$ | OH$^-$ |
| 0.245 | 0.039 | 0.058 | 0.001 | 0.002 | 0.052 | 0.458 | 4.432 | 5.122 | 0.000 |

The data processing unit 30 may calculate an effective solubility of each precipitate component using the received difference in concentration values of each ion component.

In order to calculate the effective solubility of each precipitate component, the chemical formula and solubility of each of candidate substances that may be precipitated during the concentration process may be input in advance through the thermodynamic database and the existing experimental results.

Next, the concentration experiment result data are input, and a change in concentration of each element component may be obtained through multiple non-linear regression analysis. Then, an activity coefficient of the input thermodynamic database may be corrected to calculate the effective solubility of each precipitate component. The effective solubility of each precipitate component may be calculated as a function of the ionic strength and the concentration of each ion.

As an example, the data processing unit 30 may perform the following calculations using the data obtained by the data preprocessing unit 20.

- Ionic strength $I = \frac{1}{2}\sum_i z_i^2 c_i$ = 10.706

- Calculation of $K_{SP}$ of major precipitate

$NaCl$ ($NaCl \leftrightarrow Na^+ + Cl^-$) = 22.70

$KCl$ ($KCl \leftrightarrow K^+ + Cl^-$) = 2.346

$CaSO_4 \cdot 2H_2O$ ($CaSO_4 \cdot 2H_2O \leftrightarrow Ca^{2+} + SO_4^{2-} + 2H_2O$) = 0.001387

$NaB_5O_8 \cdot 5H_2O$ ($NaB_5O_8 \cdot 5H_2O + H^+ + 2H_2O \leftrightarrow Na^+ + 5B\ OH)_3$) = $3.29 \times 10^{-6}$

$CaB_2O_4 \cdot 4H_2O$ ($CaB_2O_4 \cdot 4H_2O \leftrightarrow Ca^{2+} + 2B\ (OH)_4^-$) = $1.68 \times 10^{-4}$

$K_{SP}$ of NaCl according to the ionic strength is calculated as follows.
$K_{SP}$ of NaCl according to I

[Table 3]

| Li (g/L) | Ionic strength | [Na] | [Cl] | $K_{SP}$ |
|---|---|---|---|---|
| 1.702 | 10.706 | 4.432 | 5.122 | 22.70 |
| 1.857 | 11.102 | 4.596 | 5.312 | 24.42 |
| 2.894 | 11.826 | 4.367 | 5.536 | 24.18 |
| 3.412 | 11.983 | 4.230 | 5.609 | 23.73 |
| 5.046 | 12.495 | 3.805 | 5.84 | 22.25 |
| 9.471 | 12.636 | 3.246 | 5.898 | 19.15 |
| 11.01 | 12.604 | 3.082 | 5.874 | 18.10 |

Meanwhile, when considering $CO_2$ in the atmosphere, additional calculations may be performed considering the following reactions.

$CO_2$ (in atmosphere) $+ H_2O \leftrightarrow H_2CO_3$

$H_2CO_3 + 2\ OH^- \leftrightarrow 2H_2O + CO_3^{2-}$

$Ca^{2+} + CO_3^{2-} \rightarrow CaCO_3$

The data prediction unit 40 may apply the effective solubility value of each precipitate component received from the data processing unit 30 to predict a precipitate component, the amount of precipitates, and a concentration of an ion component in a final concentrated brine.

As an example, the data prediction unit 40 may perform the following calculations.

While gradually increasing the concentration of Li starting from 1.7 g/L received from the data collection unit 10,

1) calculate a concentration including Cl and OH
2) calculate an ionic strength
3) calculate a solubility of each precipitate
4) repeat a method of calculating 1) after removing a concentration equal to the amount of precipitates

As a result, a pH, an ion concentration, a precipitate component, and the like in the brine in each concentration stage may be predicted.

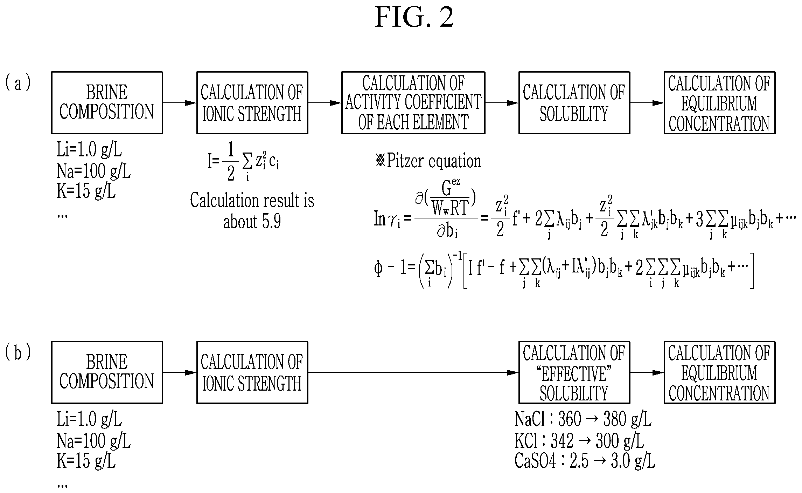

FIGS. 2A and 2B are schematic views illustrating a method of calculating component concentrations during a brine concentration process.

FIG. 2A illustrates a process of calculating concentrations of ion components in brine according to the existing thermodynamic calculation.

In general, in the existing thermodynamic calculation, a solubility product is used to calculate the solubility of precipitate, and specifically, a $pK_{SP}$ value, which is a negative log value of the solubility product, is used.

Example) In case of $CaSO_4$, when $pK_{SP} = 2.3$,

$$\frac{a_{CaSO_4}}{a_{Ca^{2+}}a_{SO_4^{2-}}} = 10^{-2.3} \quad (1).$$

In Formula (1), a is an ion activity (hereinafter, referred to as an "activity") of each component may be represented by the following relational expression.

$$a_i = \gamma_i \frac{c_i}{c^0} \quad (2)$$

Where $a_i$ is an activity of an element i, $\gamma_i$ is an activity coefficient of the element i, $c_i$ is a concentration of the element i, and co is a standard concentration and is usually 1 mol/L.

In the existing thermodynamic calculation, an ionic strength is calculated, an activity coefficient at the ionic strength corresponding to each element is calculated as an activity (concentration □× activity coefficient) according to a complex formula, and then a product of the activity and a unique solubility product of each substance are compared to calculate the presence or absence of precipitation and the amount of precipitates. Therefore, extensive calculations are required to calculate an equilibrium concentration by reflecting this.

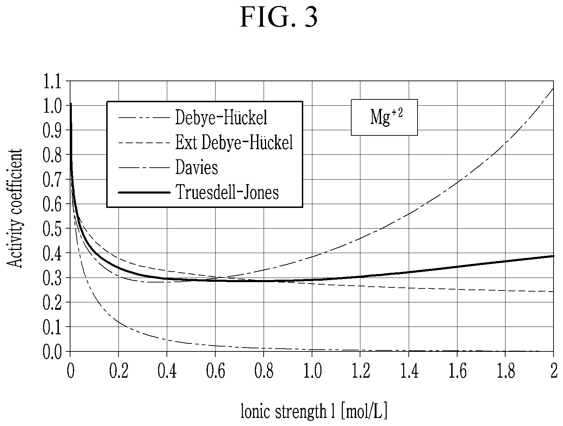

In addition, as illustrated in FIG. 3, the activity coefficient calculated from the existing thermodynamic calculation formula is known to have significant errors in solutions having a high ion concentration.

Referring to FIG. 3, it is confirmed that even when the ionic strength ( $I = \frac{1}{2}\sum_i z_i^2 c_i$ , $z_i$ is a charge of the ion and $c_i$ is a concentration of the ion) is only 2 mol/L, the error between the respective calculation formulas is severe. In particular, it is known that, in the case of brine, since the ionic strength is 5 mol/L or more, it is difficult to accurately calculate the activity coefficient using a general thermodynamic calculation formula, and even when a formula known to be well suited for a high-concentration solution is used, errors are large. When the activity coefficient is not accurate as described above, an activity value applied to the calculation of the solubility varies, and therefore, the calculated values of the solubility and the amount of the precipitates vary. Therefore, when the existing thermodynamic calculation is used to predict the brine concentration process, the amount of precipitates that differs greatly from the actual result is calculated and a solubility value that differs greatly from the actual result is applied, and as a result, the type of substance precipitated itself may vary.

In Formula (2), an activity coefficient $\gamma$ is a value that represents an effective concentration compared to the actual concentration, and in a high-concentration solution, the effective concentration may be differ from the actual concentration because a charge of ion and interaction with solvent particles are affected by other surrounding ions. The activity coefficient may be calculated using the ionic strength, which is a characteristic of the solution, and the calculation formula is as follows.

$$\ln \gamma_+ = (z_+)^2 F + \sum_a m_a \left(2B_{+,a} + ZC_{+,a}\right) + \sum_c m_c (2\Phi_{+,c} + \sum_a m_a \psi_{+,ca}) + \cdots \quad (3)$$

$$\ln \gamma_- = (z_-)^2 F + \sum_c m_c \left(2B_{c,-} + ZC_{c,-}\right) + \sum_a m_a (2\Phi_{-,a} + \sum_c m_c \psi_{-,ca}) + \cdots \quad (4)$$

In Formulas (3) and (4), the subscripts + and - are cations and anions of a substance to be considered, and a and c are cations and anions of substances other than the substance to be considered. For example, when the substance to be considered is NaCl, the superscript + means $Na^+$ and the superscript - means $Cl^-$. In addition, a may be $K^+$, $Ca^{2+}$, $Mg^{2+}$, or the like, and c may be $SO_4^{2-}$, $B(OH)_4^-$, or the like. In addition, in Formulas (3) and (4), F in the first term may be represented as follows.

$$F = -A\left[\frac{\sqrt{I}}{1+b\sqrt{I}} + \frac{2}{b}\ln(1 + b\sqrt{I})\right] \quad (5)$$

In Formulas (2) to (5), A, $B_{c,a}$, $C_{c,a}$, $\Phi_{x,y}$, $\Psi_{xc,a}$, and the like are values already reported by the Pitzer equation and the like.

FIG. 2B illustrates a process of calculating a concentration of each component in brine according to an exemplary embodiment of the present invention.

Referring to FIG. 2B, in the existing thermodynamic calculation, the presence or absence of precipitation is calculated by comparing the solubility expressed by the solubility product, which is a thermodynamic value, and the activity product of each element, but in the present invention, the amount of precipitates is calculated by comparing the corrected solubility (the solubility value is changed by calculating the activity coefficient rather than changing the activity) and the concentration of the corresponding component. Therefore, the amount of calculation is reduced, and as a result, concentration composition results that reflect actual experimental results may be obtained.

In an exemplary embodiment of the present invention, the effective solubility of the precipitate may be calculated by inputting the brine composition according to the experimental result, calculating the activity coefficient $\gamma$ of each component, calculating $K_{SP}$, and then fitting the $K_{SP}$ calculated for the target component with a linear or quadratic function of the ion concentration of each component or recalculating $K_{SP}$ of each component through machine learning.

The reason that the above calculation may be performed in the present invention is that the composition in the brine concentration process does not suddenly change significantly during the concentration process, and complex physical quantities such as the ionic strength and the activity coefficient ultimately depend on the composition, and thus, when the concentration of Na, K, or Cl changes, various thermodynamic physical quantities also change with a certain relationship. Therefore, when the solubility may be represented as a function of easily calculated ionic strength and concentration of each ion, an effective solubility suitable for the actual value may be calculated without calculating a complex activity coefficient, and thus, accuracy increases compared to the existing thermodynamic calculation, and in addition, the amount of calculation is also reduced, which has an advantage of eliminating the need for a separate system for calculation.

Meanwhile, the data collection unit 10 may collect data such as a temperature, an atmospheric pressure, and a concentration of $CO_2$ and data such as the amount of calcium carbonate ($CaCO_3$) precipitated and a pH value. High-concentration brine data may be newly collected by the data collection unit 10, and then may be transmitted to the data preprocessing unit 20. Data such as the effective solubility may be calculated by the data processing unit 30 using the results calculated by the data preprocessing unit 20. At this time, amounts of $CO_2$ absorbed and dissolved may be calculated by an equilibrium concentration calculation using the calculated and updated amount of calcium carbonate ($CaCO_3$) precipitated. The amount of carbon dioxide absorbed may be calculated using the concentrations of calcium (Ca), sulfuric acid ($SO_4$), and boron (B) in the initial brine and the high-concentration brine and the pH value. Although not illustrated in the present invention, it is obvious that the concentrations of calcium (Ca), sulfuric acid ($SO_4$), and boron (B) refer to concentrations of calcium ions, sulfuric acid groups, and boron ions.

Specifically, all changes resulting from continuous absorption of $CO_2$, which is present in a trace amount in the atmosphere, may be considered. This means that when considering the solubility of $CO_2$, the amount of $CaCO_3$ precipitated that is not previously considered is taken into consideration. Since the amount of $CaCO_3$ precipitated is 1/10 or less of the amount of $CaSO_4$ precipitated, which is a main precipitated phase of Ca, and the time it to reach the chemical equilibrium is long, these changes are not considered in most of the existing brine concentration simulation calculations. However, since Ca is converted into sulfate ($CaSO_4$), a boron compound ($CaB_xO_y$ amorphous), or the like, in addition to carbonate, when the concentration of Ca is accurately predicted, concentrations of $SO_4$ and B may also be improved.

Specifically, the reaction in which $CO_2$ in the atmosphere changes into a carbonic acid radical ($CO_3^{2-}$) may change the pH in brine as shown below.

$$CO_2(g) + H_2O(l) \rightarrow CO_3^{2-}(aq) + 2H^+(aq)$$

$CO_2$ is dissolved in the brine and releases a carbonic acid radical ($CO_3^{2-}$), and then reacts with a calcium ion and precipitates as a $CaCO_3$ precipitate, such that the carbonic acid radical ($CO_3^{2-}$) is consumed. Accordingly, $CO_2$ in the atmosphere is additionally dissolved in the brine, and in this process, the pH of the brine is lowered, and as the pH changes, the solubility of many precipitated phases changes. In particular, the solubility of $CO_2$ may play an important role in predicting a concentration of Mg because it changes the solubility of $Mg(OH)_2$, which is sensitive to pH. Therefore, the consideration of the dissolution of $CO_2$ in the atmosphere has an effect of improving the prediction of the concentrations of B, $SO_4$, and Mg, in addition to Ca.

This has an advantage of being able to more precisely predict the content of ion component and the amount of precipitates in the concentrated brine, as well as improving lithium production efficiency and economic feasibility in the entire brine concentration process.

The value of the amount of $CO_2$ absorbed is used to adjust the concentration of Ca by the data processing unit 30, and calculations are repeatedly performed until results matching the field data are produced. The results are related to the "prediction of the concentrated brine composition", and thus, the predicted composition and the amount of precipitates at the final concentration may be calculated.

Meanwhile, the data preprocessing unit 20, the data processing unit 30, and the data prediction unit 40 may repeatedly perform calculations according to a change in data of the high-concentration brine collected by the data collection unit 10 to calculate an optimal concentration of each component in the final concentrated brine, and the concentration of Li in the final concentrated brine may be 3.5 g/L to 18 g/L.

Hereinafter, exemplary embodiments of the present invention will be described in detail. However, these exemplary embodiments are provided as examples, and the present invention is not limited by these exemplary embodiments and is defined by only the scope of the claims to be described below.

(Experimental Example 1)

A concentration experiment was performed using brine. Concentrations of components at each concentration stage are shown in Table 4.

[Table 4]

| | Unit of concentration of each ion component: g/L | | | | | | |
|---|---|---|---|---|---|---|---|
| Classification | Li | SO$_4$ | Ca | Mg | B | K | Na |
| C.1 | 1.702 | 3.793 | 2.324 | 0.031 | 0.581 | 17.91 | 101.88 |
| C.2 | 1.857 | 3.763 | 2.383 | 0.049 | 0.633 | 19.35 | 106.98 |
| C.3 | 2.894 | 5.126 | 3.063 | 0.113 | 0.824 | 28.78 | 101.03 |
| C.4 | 3.412 | 5.357 | 3.119 | 0.133 | 0.848 | 33.99 | 97.80 |
| C.5 | 5.046 | 4.039 | 2.526 | 0.196 | 0.925 | 50.05 | 87.35 |
| C.6 | 9.471 | 5.839 | 2.243 | 0.356 | 0.761 | 49.69 | 69.60 |
| C.7 | 11.01 | 5.465 | 1.947 | 0.414 | 0.813 | 44.00 | 68.61 |
| C.8 | 14.40 | 6.810 | 1.960 | 0.569 | 0.883 | 52.33 | 59.76 |
| C.9 | 28.60 | 7.951 | 1.266 | 1.058 | 1.268 | 33.03 | 26.21 |

(Comparative Example 1)

Brine containing the component C.1 in Table 4 was used as a raw material, and a concentration of each ion component was calculated using the existing thermodynamics program OLI Flowsheet 11.0.

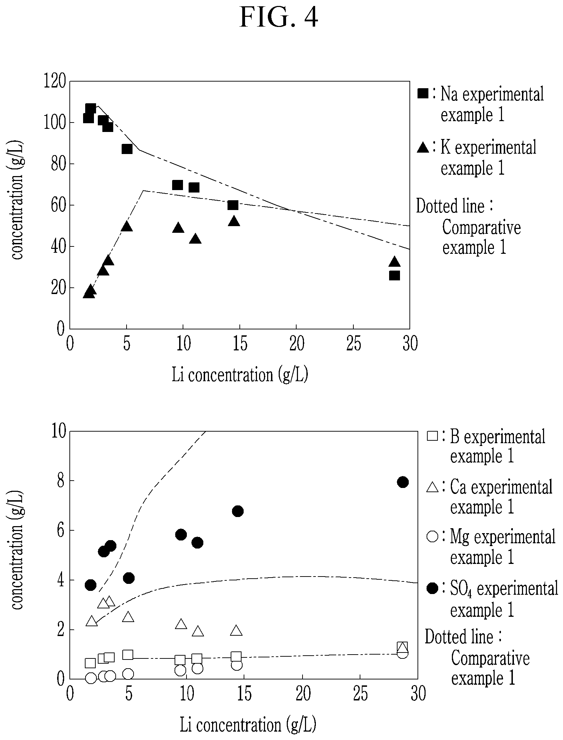

FIG. 4 is a diagram illustrating results according to Experimental Example 1 and Comparative Example 1.

Referring to FIG. 4, a difference in concentration values of each ion component between Experimental Example 1 and Comparative Example 1 is large, which is a non-ideal composition with a high concentration of ions in brine, and this is considered to be due to the difference in solubility of each ion component caused by errors occurring in the activity and solubility calculations due to the characteristics of the thermodynamics program set to allow calculations to be made in an ideal composition in a wide range.

As an example, considering a case where the amount of precipitates was calculated to be 5% or less in the error in calculating the solubility of KCl, when the concentration of Li in the brine was 1 g/L, the concentration of K was 10 g/L. As a result of performing concentration so that the concentration of Li in the brine was 25 g/L, it was assumed that the concentration of K in the concentrated brine was measured to be 45 g/L. The amount of K actually precipitated corresponds to 205 g/L after concentration. When the amount of precipitates was calculated to be 5% or less (when there were no precipitation, it should have been 10 * 25/1 = 250 g/L, but only 45 g/L remained), K equivalent to 195 g/L was predicted to be precipitated, and the concentration of K remaining was predicted to be 250 g/L - 195 g/L = 55 g/L. As a result, the error of 5% in the precipitate prediction leads to a difference of 22% in the concentrated brine composition, and the difference in concentration may affect the prediction of the amount and type of precipitates, resulting in large errors.

(Example 1)

The brine C1 in Table 4 was used as a raw material, and a concentration of each component was calculated using a brine concentration process prediction system considering the brine concentration experiment results in each stage.

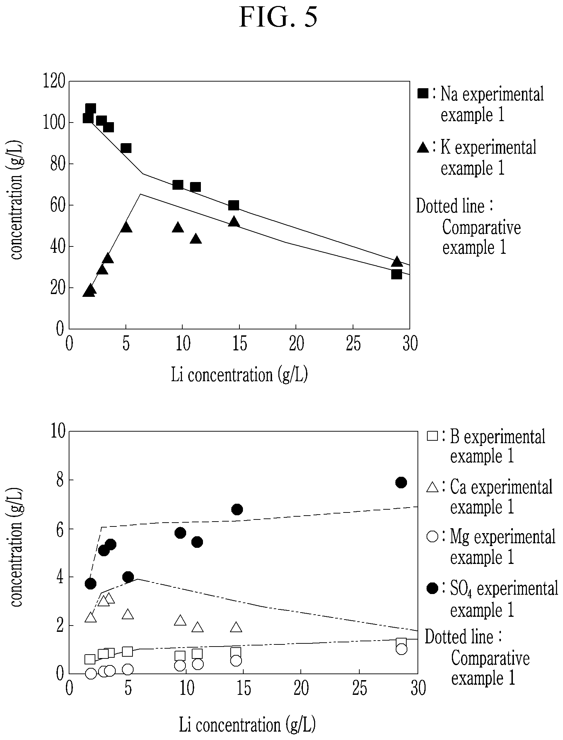

FIG. 5 is a diagram illustrating results according to Example 1 and Experimental Example 1.

Referring to FIG. 5, it can be confirmed that the difference in concentration values of each component between Example 1 and Experimental Example 1 is significantly reduced compared to Comparative Example 1 illustrated in FIG. 4. In particular, it was confirmed that the difference between Example 1 and Experiment Example 1 for the components K and $SO_4^{2-}$ was relatively reduced compared to Comparative Example 1 in FIG. 4.

(Example 2)

A concentration of each component was calculated in the same manner as that of Example 1, except that absorption of $CO_2$ was corrected in the brine concentration process prediction system.

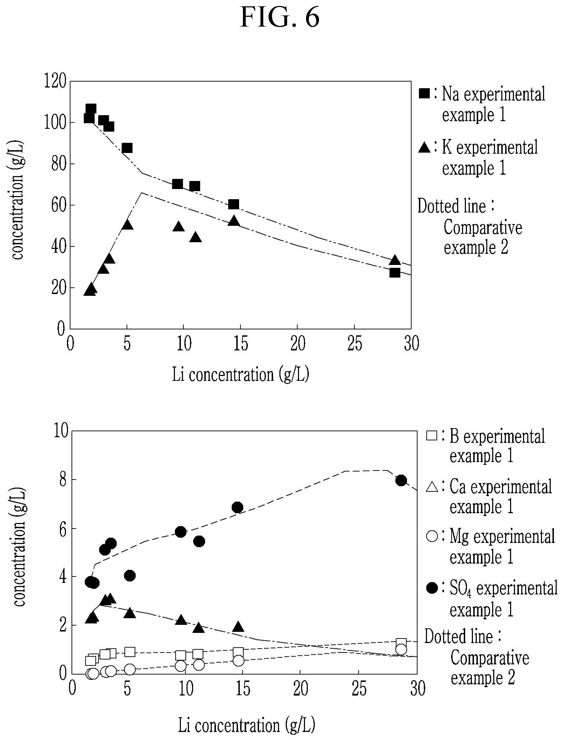

FIG. 6 is a diagram illustrating results according to Example 2 and Experimental Example 1.

Referring to FIG. 6, it can be confirmed that the difference in concentration values of each element between Example 2 and Experimental Example 1 is smaller than the difference between Example 1 and Experimental Example1, and it can be confirmed that the qualitative tendency of each element to increase or decrease depending on the concentration is also predicted similarly to the experimental results.

Meanwhile, it was confirmed that the error in the amount of precipitates generated during the concentration process according to Example 2 was 1% or less. In the present invention, 90% or more of the precipitates are (NaCl + KCl). In a case where the concentration of Li in the brine is 1.7 g/L, the concentration of Na is 101.88 g/L, when the concentration of Li and the concentration of Na in the concentrated brine are 28.6 g/L and 26.21 g/L, respectively, it is calculated that Na remains in the concentrated brine at a concentration of about 1.56 (26.21 * 1.7/28.6) g/L based on before concentration (C1 in Table 4), and a concentration of Na precipitated is calculated to be 100.3 g/L. As a result of the prediction according to Example 2, it was calculated that Na remained in the concentrated brine at a concentration of about 1.79 g/L, and 100.1 g/L of Na before concentration was precipitated as Na precipitates, which was predicted to be about 0.2% smaller than the actual experimental result (Experimental Example 1). In addition, when K precipitates were calculated using the same method, it was predicted to be 0.4% more than the actual experimental result, and the total amount of precipitates was predicted with an error range of 0.2% or less.

(Experimental Example 2)

A concentration experiment was performed using brine. Concentrations of components at each concentration stage are shown in Table 5.

[Table 5]

| Classification | Li | SO$_4$ | Ca | Mg | B | K | Na |
|---|---|---|---|---|---|---|---|
| Unit of concentration of each ion component: g/L |
| C.1 | 1.702 | 3.793 | 2.324 | 0.031 | 0.581 | 17.91 | 101.88 |
| C.2 | 1.857 | 3.763 | 2.383 | 0.049 | 0.633 | 19.35 | 106.98 |
| C.3 | 2.894 | 5.126 | 3.063 | 0.113 | 0.824 | 28.78 | 101.03 |
| C.4 | 3.412 | 5.357 | 3.119 | 0.133 | 0.848 | 33.99 | 97.80 |
| C.5 | 5.046 | 4.039 | 2.526 | 0.196 | 0.925 | 50.05 | 87.35 |
| C.6 | 9.471 | 5.839 | 2.243 | 0.356 | 0.761 | 49.69 | 69.60 |
| C.7 | 11.01 | 5.465 | 1.947 | 0.414 | 0.813 | 44.00 | 68.61 |
| C.8 | 14.40 | 6.810 | 1.960 | 0.569 | 0.883 | 52.33 | 59.76 |
| C.9 | 28.60 | 7.951 | 1.266 | 1.058 | 1.268 | 33.03 | 26.21 |

(Example 3)

The brine C1 in Table 5 was used as a raw material, and a concentration of each component was calculated in the same manner as that of Example 2.

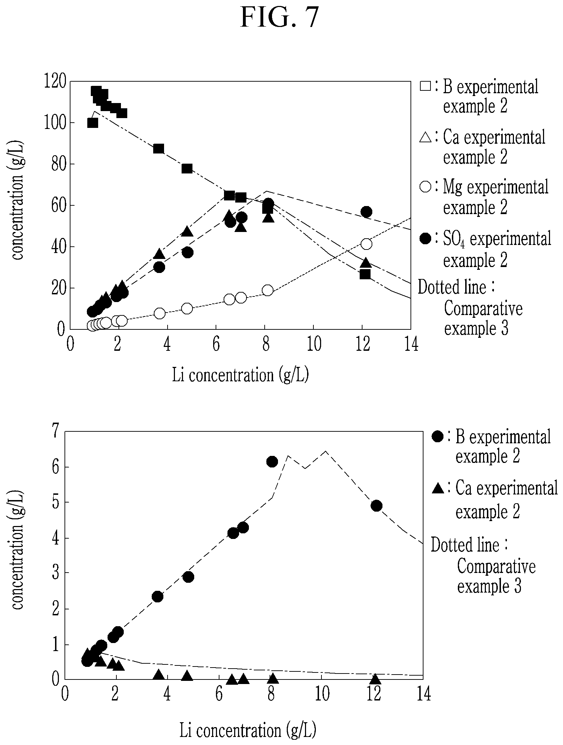

FIG. 7 is a diagram illustrating results according to Example 3 and Experimental Example 2.

Referring to FIG. 7, it can be seen that the difference in concentration values of each ion component between Example 3 and Experimental Example 2 was small. In addition, it can be seen that error ranges for Na, K, and $SO_4$ components are within 5%.

The present invention is not limited to the exemplary embodiments, but may be prepared in various different forms, and it will be apparent to those skilled in the art to which the present invention pertains that the exemplary embodiments may be implemented in other specific forms without departing from the spirit or essential feature of the present invention. Therefore, it is to be understood that the exemplary embodiments described hereinabove are illustrative rather than restrictive in all aspects.

**Claims**

1. A brine concentration process prediction system comprising:

   a data collection unit collecting data of an initial brine and a high-concentration brine;
   a data preprocessing unit converting the data collected by the data collection unit to be applied to a data processing unit provided at a later stage;
   a data processing unit calculating a solubility of each precipitate component using the data converted by the data preprocessing unit; and
   a data prediction unit predicting a final amount of precipitates and a concentration of each ion component in a final concentrated brine using the solubility of each precipitate component calculated by the data processing unit.

2. The brine concentration process prediction system of claim 1, wherein:
   the data preprocessing unit calculates a difference in concentration values of each ion component in the high-concentration brine and the initial brine collected by the data collection unit.

3. The brine concentration process prediction system of claim 1, wherein:
   the data processing unit calculating the solubility of each precipitate component using the data converted by the data preprocessing unit calculates a solubility of each precipitate component through multiple non-linear regression analysis or machine learning.

4. The brine concentration process prediction system of claim 1, wherein:
   the data processing unit additionally calculates the amount of carbon dioxide absorbed.

5. The brine concentration process prediction system of claim 2, wherein:
   the amount of carbon dioxide absorbed is calculated using concentrations of calcium (Ca), sulfuric acid ($SO_4$), and boron (B) in the initial brine and the high-concentration brine and a pH value.

6. The brine concentration process prediction system of claim 1, wherein:
   the data preprocessing unit, the data processing unit, and the data prediction unit repeatedly perform calculations according to a change in data of the high-concentration brine collected by the data collection unit to calculate an optimal concentration of each ion component in a final concentrated brine.

7. The brine concentration process prediction system of claim 6, wherein:
   a concentration of Li in the final concentrated brine is 3.5 g/L to 18 g/L.

8. The brine concentration process prediction system of claim 1, wherein:
   an error range of the final amount of precipitates predicted by the data prediction unit is 5% or less.

# FIG. 1

```
┌─────────────────────────┐
│   DATA COLLECTION UNIT   │──10
└─────────────────────────┘
             │
┌─────────────────────────┐
│  DATA PREPROCESSING UNIT │──20
└─────────────────────────┘
             │
┌─────────────────────────┐
│   DATA PROCESSING UNIT   │──30
└─────────────────────────┘
             │
┌─────────────────────────┐
│   DATA PREDICTION UNIT   │──40
└─────────────────────────┘
```

# FIG. 2

( a )

| BRINE COMPOSITION | → | CALCULATION OF IONIC STRENGTH | → | CALCULATION OF ACTIVITY COEFFICIENT OF EACH ELEMENT | → | CALCULATION OF SOLUBILITY | → | CALCULATION OF EQUILIBRIUM CONCENTRATION |
|---|---|---|---|---|---|---|---|---|

Li=1.0 g/L
Na=100 g/L
K=15 g/L
...

$$I = \frac{1}{2} \sum_i z_i^2 c_i$$

Calculation result is about 5.9

※Pitzer equation

$$\ln \gamma_i = \frac{\partial (\frac{G^{ez}}{W_w RT})}{\partial b_i} = \frac{z_i^2}{2} f' + 2\sum_j \lambda_{ij} b_j + \frac{z_i^2}{2} \sum_j \sum_k \lambda'_{jk} b_j b_k + 3\sum_j \sum_k \mu_{ijk} b_j b_k + \cdots$$

$$\phi - 1 = \left(\sum_i b_i\right)^{-1} \left[ I f' - f + \sum_j \sum_k (\lambda_{ij} + I\lambda'_{ij}) b_j b_k + 2\sum_i \sum_j \sum_k \mu_{ijk} b_j b_k + \cdots \right]$$

( b )

| BRINE COMPOSITION | → | CALCULATION OF IONIC STRENGTH | → | CALCULATION OF "EFFECTIVE" SOLUBILITY | → | CALCULATION OF EQUILIBRIUM CONCENTRATION |
|---|---|---|---|---|---|---|

Li=1.0 g/L
Na=100 g/L
K=15 g/L
...

NaCl : 360 → 380 g/L
KCl : 342 → 300 g/L
CaSO4 : 2.5 → 3.0 g/L

EP 4 636 384 A1

# FIG. 3

Ionic strength I [mol/L]

# FIG. 4

# FIG. 5

# FIG. 6

# FIG. 7

## INTERNATIONAL SEARCH REPORT

| International application No. |
|---|
| **PCT/KR2023/019732** |

| A. | CLASSIFICATION OF SUBJECT MATTER |
|---|---|

**G01N 7/04**(2006.01)i; **G01N 1/40**(2006.01)i; **G06N 20/00**(2019.01)i

According to International Patent Classification (IPC) or to both national classification and IPC

| B. | FIELDS SEARCHED |
|---|---|

Minimum documentation searched (classification system followed by classification symbols)

G01N 7/04(2006.01); C02F 1/467(2006.01); G01N 21/35(2006.01); G01N 21/59(2006.01); G01N 21/77(2006.01); G01N 31/00(2006.01); G01N 33/02(2006.01); G01N 9/00(2006.01); G01N 9/24(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 염수(salt water), 농도(concentration), 예측(prediction), 데이터(data)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y<br><br>A | JP 07-140133 A (JAPAN TOBACCO INC.) 02 June 1995 (1995-06-02)<br>    See paragraphs [0016]-[0027] and [0052]-[0054] and figures 1-4. | 1-4,6<br><br>5,7-8 |
| Y | KR 10-2249269 B1 (JTECH CO., LTD.) 10 May 2021 (2021-05-10)<br>    See paragraph [0033] and claim 1. | 1-4,6 |
| Y | JP 08-505218 A (CENTRE NATIONAL DE LA RECHERCHE SCIENTIFIQUE) 04 June 1996 (1996-06-04)<br>    See page 3 and claim 1. | 4 |
| A | JP 08-240527 A (JAPAN TOBACCO INC.) 17 September 1996 (1996-09-17)<br>    See paragraphs [0009]-[0065]. | 1-8 |
| A | JP 07-140120 A (JAPAN TOBACCO INC.) 02 June 1995 (1995-06-02)<br>    See paragraph [0009]. | 1-8 |

| ☐ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **19 March 2024** | **19 March 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

# EP 4 636 384 A1

| INTERNATIONAL SEARCH REPORT | | | | International application No. | | | |
|---|---|---|---|---|---|---|---|
| Information on patent family members | | | | **PCT/KR2023/019732** | | | |

| Patent document cited in search report | | | Publication date (day/month/year) | Patent family member(s) | | | Publication date (day/month/year) |
|---|---|---|---|---|---|---|---|
| JP | 07-140133 | A | 02 June 1995 | JP | 2517875 | B2 | 24 July 1996 |
| KR | 10-2249269 | B1 | 10 May 2021 | CN | 114839166 | A | 02 August 2022 |
| | | | | JP | 2022-118709 | A | 15 August 2022 |
| | | | | WO | 2022-169189 | A1 | 11 August 2022 |
| JP | 08-505218 | A | 04 June 1996 | AT | E182986 | T1 | 15 August 1999 |
| | | | | CA | 2109809 | A1 | 14 October 1993 |
| | | | | DE | 69325870 | T2 | 20 April 2000 |
| | | | | EP | 0592632 | A1 | 20 April 1994 |
| | | | | EP | 0592632 | B1 | 04 August 1999 |
| | | | | FR | 2689635 | A1 | 08 October 1993 |
| | | | | FR | 2689635 | B1 | 01 July 1994 |
| | | | | NO | 934360 | L | 21 January 1994 |
| | | | | WO | 93-20431 | A1 | 14 October 1993 |
| JP | 08-240527 | A | 17 September 1996 | None | | | |
| JP | 07-140120 | A | 02 June 1995 | JP | 2672068 | B2 | 05 November 1997 |

Form PCT/ISA/210 (patent family annex) (July 2022)

**EP 4 636 384 A1**

**Patent documents cited in the description**

- KR 1020220176061